# FASCICULE DE BREVET EUROPEEN

(11) **EP 2 965 745 B1**
(45) Date de publication et mention de la délivrance du brevet: **30.08.2017**
(21) Numéro de dépôt: 15175434.8
(22) Date de dépôt: 06.07.2015
(51) Int. Cl.: A61K 8/73, A61Q 19/00, A61K 8/97, A61K 31/732, A61K 36/23, A61P 17/02, A61K 45/06

(54) **COMPOSITION COSMÉTIQUE OU PHARMACEUTIQUE À APPLICATION TOPIQUE COMPRENANT UNE ASSOCIATION DE PECTINE ET D'UN EXTRAIT DE CENTELLA ASIATICA ET APPLICATIONS**
KOSMETISCHE ODER PHARMAZEUTISCHE ZUSAMMENSETZUNG ZUR LOKALEN ANWENDUNG, DIE EINE VERBINDUNG AUS PEKTIN UND EINEM EXTRAKT VON CENTELLA ASIATICA ENTHÄLT, UND IHRE VERWENDUNGEN
COSMETIC OR PHARMACEUTICAL COMPOSITION FOR TOPICAL APPLICATION COMPRISING A COMBINATION OF PECTIN AND CENTELLA ASIATICA EXTRACT AND APPLICATIONS

(30) Priorité: 07.07.2014 FR 1456538
(43) Date de publication de la demande: 13.01.2016
(73) Titulaire: Laboratoires Rivadis SAS, 79100 Louzy (FR)
(72) Inventeur: GARCIA, Cédric, 13700 MARIGNANE (FR); LARNAUDIE, Nathalie, 79100 THOUARS (FR); OLLIVIER, Evelyne, 13190 ALLAUCH (FR)
(74) Mandataire: Hervouet-Malbec, Sylvie

(56) Documents cités:
- WO-A2-00/13693
- FR-A1- 2 781 156
- FR-A1- 2 888 114
- JP-A- 2002 226 378
- DATABASE GNPD [Online] MINTEL; juillet 2009 (2009-07), Acqua di Parma: "Revitalizing Bath & Shower Oil", XP002736676, Database accession no. 1146919
- DATABASE GNPD [Online] MINTEL; mars 2010 (2010-03), "Hyper-Hydrating Anti-Wrinkle Rich Cream", XP002736677, Database accession no. 1292709
- JURAIPORN SOMBOONWONG ET AL: "Wound healing activities of different extracts of Centella asiatica in incision and burn wound models: an experimental animal study", BMC COMPLEMENTARY AND ALTERNATIVE MEDICINE, BIOMED CENTRAL LTD., LONDON, GB, vol. 12, no. 1, 20 juillet 2012 (2012-07-20), page 103, XP021121119, ISSN: 1472-6882, DOI: 10.1186/1472-6882-12-103
- WANG X-S ET AL: "Structure and potential immunological activity of a pectin from Centella asiatica (L.) Urban", CARBOHYDRATE RESEARCH, PERGAMON, GB, vol. 338, no. 22, 31 octobre 2003 (2003-10-31), pages 2393-2402, XP004467569, ISSN: 0008-6215, DOI: 10.1016/S0008-6215(03)00380-X

## Description

La présente invention se rapporte au domaine technique général des produits cosmétiques ou pharmaceutiques à usage topique, utilisables notamment sur la peau.

Plus précisément, la présente invention est relative à une composition cosmétique ou pharmaceutique à application topique comprenant, dans un milieu cosmétiquement ou pharmaceutiquement acceptable, une association de pectine et d'un extrait de *Centella asiatica,* à l'utilisation d'une telle composition pour empêcher ou réduire l'adhésion des microorganismes à la surface de la peau et/ou des muqueuses, à un procédé cosmétique non thérapeutique de soin de la peau mettant en oeuvre une telle composition cosmétique, et l'utilisation d'une telle composition pharmaceutique dans la prévention et/ou le traitement des lésions cutanées, et en particulier dans la prévention et/ou le traitement des escarres et des lésions ischémiques.

La peau humaine est un organe complexe comprenant plusieurs couches intégrées, allant de la couche superficielle, l'épiderme, jusqu'aux couches plus profondes, le derme et l'hypoderme, et chacune de ces couches possède des propriétés spécifiques permettant à l'ensemble de réagir et de s'adapter aux conditions de son environnement.

La peau humaine est peuplée en permanence d'une multitude de micro-organismes différents (bactéries, levures et champignons). La flore microbienne résidente, indispensable à la bonne santé de la peau est constituée principalement de staphylocoques (*epidermidis, hominis*), de corynebactéries, de propionibactéries Gram + ainsi que d'une flore fongique. La plupart des bactéries de la peau sont présentes sur l'épiderme squameux superficiel, colonisant les cellules mortes et étroitement associées aux glandes sébacées et sudoripares. Les excrétions de ses glandes fournissent l'eau, acides aminés, urée, électrolytes...tous ces éléments permettent une macération favorisant le développement bactérien (notamment des staphylocoques).

Les infections cutanées sont le plus souvent dues à la rupture de l'équilibre écologique de la flore résidente suite à la colonisation de la peau par des germes exogènes pathogènes ou à la prolifération anormale d'une souche endogène.

Dans le cadre hospitalier, l'infection bactérienne est un sujet très préoccupant et une lutte sans cesse pour les professionnels de santé. Ces infections nosocomiales, si elles sont absentes lors de l'admission du patient à l'hôpital se développent 48 heures au moins après l'admission. Le staphylocoque aureus, un des principaux agents pathogènes dans les hôpitaux, peut provoquer des inflammations, abcès, boutons, folliculites, impétigo, furoncles, aggravant ainsi les lésions. Cette bactérie est également présente au niveau des escarres à partir du stade 1 et contribue à son évolution. Les sujets les plus affectés sont les personnes qui ont une peau fragilisée (personne âgées, diabétiques...). Une mauvaise hygiène corporelle du patient peut avoir de graves conséquences et ainsi favoriser l'apparition d'une escarre.

Pour combattre ces micro-organismes, il est courant d'utiliser des antibiotiques tels que la méticilline ou des bactéricides. L'utilisation de ces composés pose cependant le problème de la non spécificité d'action visant indifféremment la flore pathogène et la flore résidente. Il est aussi à noter que dans le cadre hospitalier la résistance du staphylocoque aureus à la méticilline qui apparaît de plus en plus chez les patients hospitalisés pose de sérieux problèmes aux professionnels de santé. De nos jours l'utilisation des antiseptiques sur les plaies chroniques est déconseillée par les professionnels de santé (sauf en cas de surinfection avérée) car il est nécessaire de respecter l'écosystème de la plaie.

C'est pourquoi, de façon alternative à l'emploi des antibiotiques ou des bactéricides, il a déjà été proposé d'appliquer des compositions topiques contenant des composés destinés à empêcher ou réduire l'adhésion des microorganismes à la surface de la peau. Les composés utilisés dans ce but peuvent par exemple être des silicones comme décrit par exemple dans la demande internationale WO 9962475, des huiles végétales comme décrit dans la demande de brevet EP-1 133 979, ou bien encore des hydrates de carbone ou des dérivés d'hydrates de carbone comme décrit par exemple dans la demande internationale WO 9623479 qui cite de nombreux monosaccharides tels que par exemple les pentoses et les hexoses (e.g. furanose et pyranose), les disaccharides tels que le sucrose, le maltose et le lactobiose, les oligosaccharides composés de 2 à 7 oses, les amino-sucres tels que la N-acétylglucosamine et la N-galactosylamine, les glycolipides tels que les céramides, les cérébrosides et les gangliosides, les polysaccharides naturels ou synthétiques tels que l'amidon, la cellulose, les dextranes, l'inuline, la chitine, (en particulier le chitosan, l'acide alginique et les alginates), les gommes végétales, les pectines, les mannanes, les galactanes, les xylanes, les sulfates de chondroïtine, l'héparine, l'acide hyaluronique, les glycosaminoglycanes, etc ...

Enfin, la demande de brevet FR 2 888 114 propose d'utiliser une composition comprenant une association de madécassoside et de terminoloside afin de réduire l'adhésion des microorganismes à la surface de la peau et/ou des muqueuses.

Bien que présentant un intérêt dans la mesure où il s'agit pour la plupart de composés d'origine naturelle, l'utilisation de ces hydrates de carbone ne donne pas toujours satisfaction en terme d'efficacité et il subsiste donc un besoin pour des compositions présentant des propriétés améliorées vis-à-vis de la réduction ou de la suppression de l'adhésion des microorganismes à la surface de peau et/ou des muqueuses. Il est en particulier recherché que la composition soit à base d'un ou de deux principes actifs naturels (non synthétiques), et qu'elle n'engendre pas de déséquilibre de la flore microbienne naturelle de la peau tout en empêchant la colonisation de la peau par des germes exogènes pathogènes ou à la prolifération anormale d'une souche endogène.

Par ailleurs, on peut également citer les documents XP002736676 (Revitalizing Bath & Shower Oil) et XP002736677 (Hyper-Hydrating Anti-Wrinkle Rich Cream), qui décrivent des compositions cosmétiques à base de pectine et d'un extrait de Centella asiatica.

Les études effectuées par la société demanderesse ont maintenant mis en évidence que l'association d'au moins une pectine et d'au moins un extrait de *Centella asiatica* présentait un effet de synergie vis-à-vis de la réduction ou de la suppression de l'adhésion des microorganismes à la surface de la peau lorsque cette association était appliquée de façon topique et que ces propriétés pouvaient avantageusement être mises à profit pour la préparation d'une composition cosmétique et/ou pharmaceutique à application topique. L'effet anti-adhésion de ladite association est en effet supérieur aux effets obtenus à concentrations équivalentes avec chacun des composés testé individuellement à la même concentration. De plus, l'effet de synergie existant entre la pectine et l'extrait de *Centella asiatica* permet d'obtenir une inhibition de l'adhésion de l'ordre de 96 % en mettant en oeuvre une composition contenant 0,1 % en masse de pectine et 0,5 % en masse d'extrait de *Centella asiatica,* alors que l'application de compositions ne contenant que de la pectine (à raison de 0,1 % massique) ou que l'extrait de *Centella asiatica* (à raison de 0,5 % massique), n'induit respectivement que 64 % et que 60,2 % d'inhibition de l'adhésion. Ainsi, les essais effectués par la société demanderesse montrent qu'il faut utiliser 10 fois plus de pectine seule (1 % en masse) pour obtenir une inhibition de l'adhésion équivalente à celle qui est obtenue lorsque la pectine est associée à l'extrait de *Centella asiatica.*

Cet effet de synergie est d'autant plus surprenant qu'il n'est pas observé lorsque l'extrait de *Centella asiatica* est utilisé en association avec l'acide galacturonique monomère des oligosaccharides pectiques ou de la pectine.

La présente invention a donc pour premier objet une composition cosmétique ou pharmaceutique à application topique, telle que définie dans la revendication 1.

Les pectines sont des hétéropolysaccharides qui se présentent sous la forme de polymères linéaires d'acide α-D-galacturonique joints en 1-4 par une liaison glycosidique. Elles sont composées de différents polysaccharides associés : les homogalacturonanes, les xylogalacturonanes, les rhamnogalacturonanes, les arabinanes, les galactanes et les arabinogalactanes. (Perrone P et al., Phytochemistry, 2002, 60, 67-77). Par ailleurs, les fonctions carboxyliques présentes en position 6 des acides galacturoniques peuvent être estérifiées à différents degrés par des groupements méthyle. Les pectines sont de plus en plus utilisées dans le domaine de la cosmétique, principalement à titre d'agent épaississant de milieux aqueux. Elles sont de plus en plus appréciées pour leurs propriétés stabilisantes, gélifiantes et pour leur biodégradabilité. Elles sont aussi utilisées comme support pour l'administration de médicaments dans le tractus gastro-intestinal, tels que des comprimés matriciels ou des billes de gel ou comme prébiotique.

La ou les pectines utilisables selon l'invention peuvent être extraites de différents végétaux parmi lesquels figurent principalement les fruits tels que la pomme (pulpe et peau) et les agrumes, en particulier le citron, ainsi que le tournesol et la betterave.

Les pectines utilisables selon la présente invention ont de préférence un degré d'estérification (% en nombre de fonctions carboxyliques des unités acide galacturonique estérifiées par des groupes méthyle) variant entre 65 et 80 %, et encore plus préférentiellement de 72 à 76 % environ.

Selon une forme de réalisation préférée de l'invention, la pectine est une pectine de pomme telle que par exemple la pectine vendue par la société Herbstreith & Fox sous la dénomination commerciale Pectin Classic AU 201 USP. Cette pectine présente un pH de 2,8 ± 0,2 en solution à 2,5 % en masse en solution dans de l'eau distillée à 20°C, ainsi qu'un degré d'estérification en groupement méthyle d'environ 72 à 76 %. Elle est par ailleurs référencée CAS N° 5000-69-5 au Chemical Abstracts.

*Centella asiatica* est une espèce de plante herbacée annuelle de la famille des Apiaceae originaire d'Asie et d'Océanie. Elle est utilisée comme plante médicinale dans la médecine ayurvédique et la médecine traditionnelle chinoise. Elle était connue également sous le nom scientifique de *Hydrocotyl asiatica L.* et différents noms vernaculaires, Gotu kola, Antanan, Pegaga, et Brahmi (ce dernier nom étant utilisé pour différentes espèces dont Bacopa monnieri). En médecine ayurvédique, elle est encore dénommée « l'herbe du tigre » car ces derniers avaient pour habitude de se rouler dans ces herbes pour soigner leurs blessures. En effet, cette plante, en usage externe, améliore le processus de cicatrisation en stimulant la production de collagène et aussi de fibroblastes, des cellules qui permettent la régénération des tissus. Elle agit également sur les brûlures, sur la guérison des plaies, sur les cicatrices post chirurgicales, les inflammations de la peau et le psoriasis. Les extraits de *Centella asiatica* comprennent des hétérosides (principalement asiaticoside et madécassoside) qui sont des complexes sucrés qui constituent respectivement les formes de réserve de l'acide asiatique et de l'acide madécassique de la plante.

L'extrait de *Centella asiatica* utilisable selon l'invention est de préférence un extrait sec. Il peut être obtenu à partir de la plante entière ou des parties aériennes de la plante après séchage et éventuellement broyage (tiges, feuilles), par macération à l'aide d'un solvant aqueux tel que l'eau ou hydroalcoolique tel que par exemple l'éthanol à 50°.

Parmi les extraits de *Centella asiatica* utilisables selon l'invention, on peut en particulier citer l'extrait sec vendu par la société Cooper et dont la composition est la suivante : Stérols, hétérosides de flavonols, de coumarines telle l'esculétine, de Polyines et saponosides : asiaticosides et madécassoside qui sont des esters d'un trisaccharide et d'acides triterpéniques que sont les acides asiatiques et madécassique.

La composition cosmétique ou pharmaceutique comprend de 0,05 à 0,5 % en masse environ de pectine et de 0,1 à 1 % en masse environ d'extrait de *Centella asiatica* par rapport à la masse totale de la composition.

En effet, les essais effectués par la société demanderesse ont montré que l'effet inhibiteur de l'adhésion des microorganismes de la pectine était le plus élevé à une concentration voisine de 0,1 % en masse lorsque celle-ci est associée à un extrait de *Centella asiatica* à raison d'environ 0,5 % en masse par rapport à la masse totale de la composition.

Selon la présente invention, on entend par « support cosmétique ou pharmaceutiquement acceptable » tout mélange de solvants et d'excipients habituellement utilisés pour la préparation de compositions cosmétiques ou pharmaceutique, ces solvants et excipients étant bien entendu compatible avec une application sur la peau ou les muqueuses.

Ainsi le support cosmétiquement ou pharmaceutiquement acceptable peut être constitué d'eau ou d'un mélange d'eau et d'au moins un solvant organique physiologiquement acceptable tel que l'éthanol, l'isopropanol, le propanol, le butanol, les polyéthylèneglycols ayant par exemple de 6 à 80 motifs d'oxyde d'éthylène comme le propylène glycol, l'isoprène glycol, le butylène glycol, le glycérol et le sorbitol.

La composition cosmétique ou pharmaceutique conforme à la présente invention présente un pH compatible avec la peau, variant de préférence de 3 à 8, et encore plus préférentiellement de 4,8 à 6,8.

Selon une forme de réalisation préférée, la composition cosmétique ou pharmaceutique conforme à l'invention comprend en outre au moins une phase grasse, apportant du confort, de la douceur et permettant également de relipider la peau.

Cette phase grasse peut comporter une ou plusieurs huiles choisies de préférence dans le groupe constitué par :
- les silicones volatiles ou non volatiles, linéaires, ramifiées ou cycliques, organo-modifiées ou non, hydrosolubles ou liposolubles parmi lesquelles on peut notamment citer à titre d'example a diméthicone, le cyclopentasiloxane et l'octaméthylcyclotétrasiloxane ;
- les huiles minérales telles que par exemple l'huile de vaseline et la paraffine ;
- les huiles animales telles que par exemple le perhydrosqualène ;
- les huiles synthétiques telles que par exemple les isoparaffines, le dipélargonate de propylène glycol, le néopentanoate d'isostéaryle et le myristate d'isopropyle ;
- les esters d'acides gras tels que par exemple le caprylate caprate de coprah, le palmitate d'éthylhexyle, le palmitate d'isopropyle ;
- les huiles fluorées et perfluorées telles que par exemple les perfluoroalcanes ;
- des matières grasses supplémentaires tels que des cires comme par exemple la cire de carnauba, la cire d'abeille, des acides gras tels que l'acide stéarique, l'acide palmitique, ou bien encore des alcools gras tels que l'alcool cétylique, l'alcool stéarique, l'alcool cétéarique, l'alcool laurique et l'alcool myristique.

Dans ce cas, la phase grasse représente de préférence de 1 à 80 % en masse environ, et encore plus préférentiellement de 20 à 40 % en masse environ par rapport à la masse totale de la composition.

Les compositions cosmétiques ou pharmaceutiques conformes à la présente invention sont compatibles avec la peau et éventuellement avec les lèvres, le cuir chevelu, les cils, les yeux et/ou les cheveux. Ces compositions sont donc physiologiquement acceptables. De plus, les compositions cosmétiques ou pharmaceutiques de la présente invention possèdent une excellente tolérance cutanée, notamment en raison du film lipidique résultant de la présence préférentielle d'une phase grasse, elles ne présentent aucune phototoxicité et leur application sur la peau, pour des périodes de temps prolongées, n'implique aucun effet systémique.

La composition cosmétique ou pharmaceutique conforme à l'invention peut se présenter sous toutes les formes galéniques normalement utilisées pour une application par voie topique, en particulier sous forme de produits liquides, pâteux, semi-solides, anhydres ou solides comme les patchs, les lotions, les eaux micellaires, les gels aqueux, les gel huileux, les émulsions huile-dans-eau, eau-dans-huile, ou multiples (eau-huile-eau ou huile-eau-huile) ou les dispersions d'huile dans une phase aqueuse à l'aide de sphérules, de nanoparticules polymériques, les nano-émulsions, et les microémulsions.

Les gels huileux peuvent en particuliers se présenter sous la forme de gels huileux de type D-phase qui sont des émulsions huile-dans-eau (H/E) contenant une large proportion de phase grasse (de 40 à 95% en masse), offrant l'aspect d'une microémulsion gélifiée transparente. L'ajout d'eau dans un gel huileux de type D-Phase entraîne une dilution et une sortie de la zone de transparence. Les émulsions H/E obtenues sont alors très fines, très stables et présentent une viscosité modulable à volonté pour créer des produits allant de la crème au spray.

Ces formes galéniques sont préparées par les techniques usuelles, et par exemple, dans le cas d'une crème, par dispersion d'une phase grasse dans une phase aqueuse pour obtenir une émulsion huile-dans-eau, ou inversement pour préparer une émulsion eau-dans-huile.

Selon un mode préféré de réalisation de l'invention, la composition cosmétique ou pharmaceutique est sous forme d'une émulsion huile-dans-eau.

En plus de la ou des pectines et de l'extrait de *Centella asiatica,* la composition cosmétique ou pharmaceutique conforme à l'invention peut comprendre en outre un ou plusieurs principes actifs secondaires complétant avantageusement leur activité, et compatibles, c'est-à-dire non susceptibles de réagir les uns sur les autres ou de masquer ou limiter leurs effets respectifs.

Parmi de tels principes actifs secondaires, on peut notamment mentionner les agents desquamants, les agents hydratants, les agents anti-séborrhéiques, les agents dépigmentants ou propigmentants, les agents anti-glycation, les inhibiteurs de la NO-synthase, les inhibiteurs de la 5α-réductase, les agents stimulant la synthèse de macromolécules dermiques ou épidermiques et/ou empêchant leur dégradation tels que par exemple la superoxyde dismutase, la carnosine, et l'aminoguanidine, les agents stimulant la prolifération des fibroblastes ou des kératinocytes et/ou la différenciation des kératinocytes, les agents myorelaxants, les agents tenseurs, les agents anti-pollution ou anti-radicalaires, les agents apaisants, les actifs lipolytiques, ainsi que les agents agissant sur la microcirculation ou sur le métabolisme des cellules.

De façon connue, la composition cosmétique ou pharmaceutique selon la présente l'invention peut en outre renfermer un ou plusieurs excipients habituels dans le domaine pharmaceutique tels que des solvants, des gélifiants et/ou épaississants classiques hydrophiles ou lipophiles ; des conservateurs ; des antioxydants ; des parfums ; des émulsionnants, des agents hydratants, des agents pigmentants ; des dépigmentants ; des agents de pénétration ; des agents hydratants, des agents kératolytiques ; des vitamines ; des émollients ; des séquestrants ; des tensio-actifs ; des polymères ; des ajusteurs de pH, des agents anti-radicaux libres ; des céramides ; des filtres solaires (chimiques ou physiques) ; des répulsifs pour insectes ; des agents amincissants ; des matières colorantes ; des antipelliculaires, etc...

Parmi les tensio-actifs, on peut en particulier citer les tensio-actifs siliconés, les esters d'acides gras et de polyols.

La composition cosmétique ou pharmaceutique selon la présente invention permet de réduire ou de supprimer l'adhésion des microorganismes à la surface de la peau.

Dans le domaine cosmétique, il est ainsi possible d'utiliser la composition cosmétique conforme à l'invention pour le nettoyage, le démaquillage et/ou les soins de la peau, ou bien encore à titre de déodorant ou pour les soins d'hygiène corporelle. En effet, la flore microbienne présente à la surface de la peau est responsable d'un grand nombre de désordres (odeurs, boutons, etc ...).

Ainsi, l'invention a également pour objet l'utilisation d'une composition cosmétique à application topique telle que définie précédemment, c'est-à-dire comprenant, dans un support cosmétiquement acceptable, au moins une pectine en une quantité variant de 0,05 à 0,5 % en masse environ par rapport à la masse totale de la composition et au moins un extrait de *Centella asiatica* en une quantité variant de 0,1 à 1 % en masse environ par rapport à la masse totale de la composition, pour le nettoyage, le démaquillage et/ou les soins de la peau, à titre de déodorant ou pour les soins d'hygiène corporelle.

Selon l'invention, l'expression « soins d'hygiène corporelle » englobe l'application de la composition cosmétique conforme à l'invention, sur les différentes parties superficielles du corps humain, y compris la cavité buccale et les muqueuses, en vue de les nettoyer, de les protéger, de les conserver en bon état ou d'en corriger ou modifier l'odeur.

Un autre objet de l'invention est un procédé cosmétique non thérapeutique de nettoyage et/ou de démaquillage de la peau comprenant l'application topique d'une composition cosmétique telle que définie précédemment, c'est-à-dire d'une composition cosmétique à application topique comprenant, dans un support cosmétiquement acceptable, au moins une pectine en une quantité variant de 0,05 à 0,5 % en masse environ par rapport à la masse totale de la composition et au moins un extrait de *Centella asiatica* en une quantité variant de 0, 1 à 1 % en masse environ par rapport à la masse totale de la composition, sur les zones de la peau concernées.

Enfin, la composition pharmaceutique conforme à l'invention et telle que définie précédemment, peut avantageusement être utilisée dans la prévention et/ou le traitement des affections cutanées impliquant la présence de microorganismes à la surface de la peau, en particulier pour la prévention et/ou le traitement des escarres et des lésions ischémiques.

En effet, les essais effectués par la société Demanderesse ont également mis en évidence que la pectine présente, lorsqu'elle est appliquée de façon topique, des propriétés anti-inflammatoires et cicatrisantes. Ainsi, les propriétés d'inhibition de l'adhésion des microorganismes sur la peau de l'association de la pectine et de l'extrait de *Centella asiatica,* combinées aux propriétés des pectines (anti-inflammatoires et cicatrisantes) font que la composition pharmaceutique utilisée selon l'invention est particulièrement bien adaptée à la prévention et/ou au traitement de ces pathologies.

C'est pourquoi, l'invention a également pour objet une composition pharmaceutique à application topique comprenant, dans un support pharmaceutiquement acceptable, au moins une pectine en une quantité variant de 0,05 à 0,5 % en masse environ par rapport à la masse totale de la composition et au moins un extrait de *Centella asiatica* en une quantité variant de 0, 1 à 1 % en masse environ par rapport à la masse totale de la composition, pour une utilisation dans la prévention et/ou le traitement des affections cutanées impliquant la présence de microorganismes à la surface de la peau, en particulier la prévention et/ou le traitement des escarres et des lésions ischémiques.

Selon cette utilisation, l'application de la composition pharmaceutique est de préférence réalisée par effleurage des zones de la peau concernées.

Les exemples suivants illustrent l'invention plus en détail sans en limiter la portée. Dans tous les exemples de compositions qui suivent, les parties sont exprimées en masse, sauf indication contraire.

### EXEMPLES

### EXEMPLE 1 : Mise en évidence de l'effet de synergie entre la pectine et l'extrait de Centella asiatica sur les propriétés anti-adhésion

Dans cet exemple, on a testé et comparé les propriétés anti-adhésion vis-à-vis des microorganismes de la pectine seule (E440) vendue sous la dénomination commerciale Pectin Classic AU 201 USP par la société Herbstreith & Fox, de l'extrait de *Centella asiatica* seul vendu sous la référence *Centella asiatica ES* par la société Cooper, et de l'association de ces deux composés.

### 1.1 Principe du test et protocole

Le test d'adhésion des agents pathogènes (microorganismes) permet d'évaluer les capacités d'un actif à empêcher l'adhésion d'une souche virulente de Staphylocoque doré sur des cornéocytes prélevés chez un donneur volontaire. Il consiste à prélever les cornéocytes au moyen d'un disque collant et de mettre ces cornéocytes en contact avec une suspension bactérienne (*Staphylococcus aureus CIP65-8)* en présence du ou des actifs à tester. Après un temps de contact d'une heure et un lavage, le tapis cellulaire est photographié au microscope et les bactéries ayant adhéré sur ce tapis cellulaire sont comptabilisées.

Les cornéocytes proviennent d'un donneur volontaire ne présentant aucun signe clinique de pathologie infectieuse, sans traitement antibiotique, ni de troubles dermatologiques. Ils ont été prélevés par décollement au moyen de disques adhésifs au niveau des avant-bras.

L'extrait de *Centella asiatica* a été solubilisé dans le diméthylsulfoxyde (DMSO ; Sigma Aldrich). La pectine a été solubilisée dans du tampon phosphate salin (PBS ; Sigma Aldrich) et chauffée à 80°C pendant 30 minutes.

Les images des bactéries adhérentes ont été acquises à l'aide d'un microscope Olympus® BX 53 (Olympus France) couplé à une caméra numérique Luca S (Andor Technology, Belfast, Ireland) et piloté par le logiciel Andor Komet (version 6.0). Le grossissement était de x400. Les images au format JPEG ont ensuite été analysées par le logiciel UTHSCSA Image Tool (version 3.0, Reindeer Graphics Inc., Asheville NC, USA). Le compte final est la moyenne arithmétique de 20 images ce qui correspond à la surface de 200 cornéocytes.

Pour chaque élément d'essai solubilisé dans le DMSO ou dans le PBS, une solution mère (solution Stock) a été préparée, filtrée à 22 µM et conservée à 4°C dans l'obscurité pendant 24 heures. Les concentrations préparées pour les solutions mères étaient les suivantes :
- *Centella asiatica* : 10% dans le DMSO
- Pectine de pomme: 1% dans le PBS.

La croissance bactérienne a été évaluée par mesure de la densité optique à 650 nm (longueur d'onde absorbée par la matière vivante), après agitation des plaques pendant 30 secondes.
Calculs réalisés : Pourcentage d'inhibition de l'adhésion bactérienne
   Pourcentage d'inhibition de la croissance bactérienne
Concentrations massiques testées de pectine : 0,05%, 0,1% ;
Concentrations massiques testées d'extrait de *Centella asiatica* : 0,1% et 0,5%.
Contrôle solvant : PBS, DMSO.
Contrôle positif: Rhamnose, concentration à 0,1%, pourcentage d'inhibition de l'adhésion bactérienne : 47,8%

Un essai comparatif a également été effectué avec de l'acide galacturonique seul, à 1 % et à 5 % en masse dans le PBS ou en association avec l'extrait de *Centella asiatica.*

### 1.2. Résultats

Les résultats obtenus avec les différentes concentrations massiques de pectine seule, d'extrait de *Centella asiatica* seul ou de la combinaison de ces deux composés sont reportés dans le tableau 1 ci-après :

**TABLEAU 1**

| Compositions testées | Concentration en pectine (% massique) | Concentration en extrait de *Centella asiatica* (% massique) | % d'inhibition de l'adhésion bactérienne | % d'inhibition de la croissance bactérienne |
|---|---|---|---|---|
| A⁽¹⁾ | 0,05 | - | 40 | 0 |
| B⁽¹⁾ | 0,1 | - | 64 | 0 |
| C⁽¹⁾ | - | 0,1 | 34,5 | 0 |
| D⁽¹⁾ | - | 0,5 | 60,2 | 0 |
| E | 0,05 | 0,1 | 77,2 | 0 |
| F | 0,1 | 0,1 | 84,5 | 0 |
| G | 0,05 | 0,5 | 95,4 | 0 |
| H | 0,1 | 0,5 | 95,7 | 0 |

| | | | | |
|---|---|---|---|---|
| (1) Composition comparative ne faisant pas partie de l'invention | | | | |

La composition E conforme à l'invention contenant les plus faibles concentrations de pectine et d'extrait de *Centella asiatica* (respectivement 0,05% et 0,1%) a montré le plus faible effet anti-adhésion, avec 22,8 % d'adhésion par rapport au témoin négatif, ce qui représente un taux d'inhibition de 77,2 %. Cet effet anti-adhésion est cependant très nettement supérieur à celui qui est observé avec la même concentration de pectine seule (Composition A : seulement 40 % d'inhibition) ou avec l'extrait de *Centella asiatica* seul à la même concentration (Composition C : seulement 34,5% d'inhibition).

La composition F conforme à l'invention comprenant 0,1 % massique de pectine et 0,1 % d'extrait de *Centella Asiatica* a montré un effet anti-adhésion intermédiaire, avec un taux d'adhésion de 15,4 % par rapport au témoin négatif, ce qui représente un taux d'inhibition de 84,5%. Comparativement, cet effet est également très supérieur à celui qui est observé avec la pectine utilisée seule à la même concentration (Composition A : seulement 40 % d'inhibition) ou avec l'extrait de *Centella asiatica* utilisé seul à la même concentration (Composition C : seulement 34,5% d'inhibition).

Les compositions G et H conformes à l'invention comprenant la pectine à la concentration massique de 0,05 % ou de 0,1 % et l'extrait de *Centella Asiatica* à la concentration massique de 0,5% ont montré les plus fortes activités anti-adhésion, avec des taux d'adhésion de 4,6 % et 4,2% respectivement par rapport au témoin négatif, ce qui représente des taux d'inhibition de 95,4 et 95,7 %, respectivement. Là encore, ces effet sont très supérieurs à ceux qui sont observés avec la pectine utilisée seule à la même concentration (Composition A : seulement 40 % d'inhibition et Composition B : seulement 64 % d'inhibition à la concentration massique de 0,5 %) ou avec l'extrait de *Centella asiatica* utilisé seul à la même concentration (Composition D : seulement 60,2 % d'inhibition).

Les résultats de l'essai comparatif effectué sur l'acide galacturonique seul ou en association avec l'extrait de *Centella asiatica* sont donnés dans le Tableau 2 ci-après :

**TABLEAU 2**

| Compositions testées | Concentration en acide galacturonique (% massique) | Concentration en extrait de *Centella asiatica* (% massique) | % d'inhibition de l'adhésion bactérienne | % d'inhibition de la croissance bactérienne |
|---|---|---|---|---|
| I⁽¹⁾ | 1,0 | - | 7,1 | 0 |
| J⁽¹⁾ | 5,0 | - | 7,1 | 0 |
| C⁽¹⁾ | - | 0,1 | 34,5 | 0 |
| D⁽¹⁾ | - | 0,5 | 60,2 | 0 |
| K⁽¹⁾ | 1,0 | 0,1 | 32,1 | 0 |
| L⁽¹⁾ | 1,0 | 0,5 | 37,6 | 0 |
| M⁽¹⁾ | 5,0 | 0,1 | 49,1 | 0 |
| N⁽¹⁾ | 5,0 | 0,5 | 54,5 | 0 |

| | | | | |
|---|---|---|---|---|
| (1) Composition comparative ne faisant pas partie de l'invention | | | | |

Si l'on compare l'activité anti-adhésion de *Centella asiatica* observée aux concentrations de 0,1 et 0,5% avec celles des mélanges, on remarque que les mélanges ont une activité plus faible que celle de l'extrait de *Centella asiatica* utilisé seul.

On peut donc en conclure que l'ajout de l'acide galacturonique n'améliore pas l'effet anti-adhésion l'extrait de *Centella asiatica,* et tendrait plutôt à le diminuer. En conséquence, il est possible de conclure qu'il n'y a pas d'effet de synergie entre l'extrait de *Centella asiatica* et l'acide galacturonique sur l'activité anti-adhésion, et qu'il y aurait plutôt un effet antagoniste.

Pour montrer la synergie entre la pectine et l'extrait de *Centella asiatica,* une étude sur leurs effets combinés a été réalisée. Un test anti-adhésion a donc aussi été réalisé sur des compositions comprenant d'autres concentrations massiques d'extrait de *Centella asiatica,* données indispensables pour pouvoir étudier leurs effets combinés (à 1 % : 80,3 % d'inhibition et à 2 % massique : 81,3 % d'inhibition).

Après obtention de ces données, l'étude de l'effet combiné de l'extrait de *Centella asiatica* et de la pectine sur l'activité anti-adhésion a pu être effectuée à partir de l'ensemble des résultats obtenus sur les actifs seuls et sur les mélanges, par la méthode des isobologrammes.

La concentration en actif seul ou en mélange capable de diminuer l'adhésion bactérienne de 80% (Concentration Inhibitrice 80%, CI-80) a été choisie comme base pour la construction de l'isobologramme. Pour l'extrait de *Centella asiatica,* la CI-80 est de 1%. Pour la pectine, la CI-80 a été déterminée par régression non linéaire grâce au logiciel Tablecurve® à partir de la relation dose-réponse obtenue dans une précédente étude : elle est de 0,2 %. Les valeurs des CI-80 correspondant aux différents mélanges ont été calculées à partir de projections mathématiques effectuées sur les résultats obtenus.

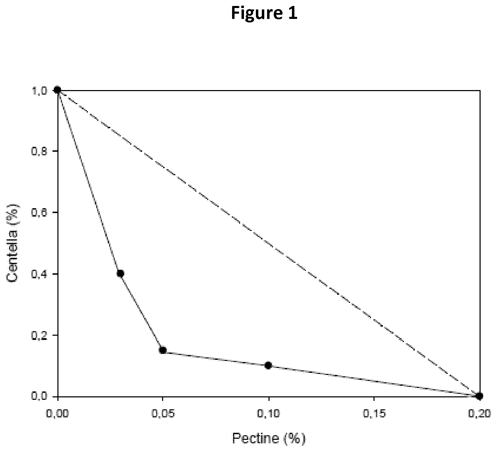

Les résultats obtenus sont représentés à la figure 1 annexée qui est l'isobologramme de l'association entre l'extrait de *Centella asiatica* et la Pectine, basé sur les concentrations induisant 80% d'inhibition de l'adhésion bactérienne. Sur cette figure, le % d'inhibition observé avec l'extrait de *Centella asiatica* est donné sur l'axe des ordonnées et le % d'inhibition observé avec la pectine est donné sur l'axe des abscisses. La ligne en pointillés représente les résultats théoriques pour un effet additif entre la pectine et l'extrait de *Centella asiatica.*

La relation dose-réponse entre les concentrations en pectine et en extrait de *Centella asiatica* représente la réponse effectivement obtenue avec les mélanges. Elle se situe bien en deçà de la ligne théorique d'un effet additif, et signifie que les deux actifs exercent une activité anti-adhésion synergique.

### Exemple 2 : Mise en évidence des propriétés anti-inflammatoire de la pectine

Dans cet exemple, on a testé les propriétés anti-inflammatoires de la pectine (E440) vendue sous la dénomination commerciale Pectin Classic AU 201 USP par la société Herbstreith & Fox.

### 2.1 Principe du test et protocole

La réponse inflammatoire globale se traduit généralement au niveau des macrophages par la production d'oxyde nitrique (NO), qui précède celle des agents pro-inflammatoires. Le test d'activité anti-inflammatoire consiste à mesurer l'inhibition de la production de NO par l'actif à tester (ici la pectine) dans des macrophages de souris préalablement activés par le lipopylosaccharide (LPS, Sigma Aldrich) d'*E. Coli.*

A titre de témoin positif, on a utilisé de la Dexaméthasone, qui est un anti-inflammatoire connu.

La concentration préparée pour la solution mère (filtrée à 22 µM et conservée à 4°C dans l'obscurité pendant 24 heures) est la suivante :
- Pectine de pomme: 1% dans le PBS (Sigma Aldrich).

Des macrophages RAW 264.7 (Sigma Aldrich) ont été transférés, à une concentration de 1,5.10⁵ cellules/ml, dans une plaque de 48 puits (1 ml/puits) et incubés à 37°C. Lorsque les cellules ont été à confluence, le milieu de culture a été changé (200 µl/puits). La pectine en solution dans le PBS a été ajoutée dans les puits, et les cellules ont été incubées pendant une heure à 37°C. Le LPS a ensuite été ajouté à la concentration de 1 µg/ml. Après addition de LPS, les cellules ont été maintenues en culture pendant 18 heures. A la fin de la période d'incubation, 50 µl de surnageant cellulaire ont été prélevés et mélangés à 50 µl de sulfanilamide (Sigma Aldrich). Après 10 minutes à 20°C à l'obscurité, 50 µl de NED (Promega) (réactif de Griess) ont été ajoutés.

L'absorbance a été mesurée à 550 nm à l'aide d'un spectrophotomètre/fluorimètre vendu sous la dénomination commerciale Infinite® M200 PRO par la société TECAN.

Concentrations massiques testées de pectine : 0,01%, 0,05%, 0,1%, 0,5%, et 1%.
Contrôle solvant : DMSO (1%, v:v)
Contrôle positif : Dexaméthasone (50 µM) : pourcentage d'inhibition du pouvoir anti-inflammatoire 72%.

### 2.2. Résultats

Les résultats, exprimés en pourcentage d'inhibition de production de NO par rapport au témoin LPS, sont présentés dans le tableau 3 ci-après.

**TABLEAU 3**

| **Concentration en pectine (% massique)** | **% d'inhibition de la production de NO** |
|---|---|
| 0,01 | 12 |
| 0,05 | 31 |
| 0,1 | 41 |
| 0,5 | 48 |
| 1 | 59 |

Ces résultats montrent que la pectine présente une activité anti-inflammatoire avec une diminution de 59% de la production de NO à la concentration de 1%.

### EXEMPLE 3 : Mise en évidence des propriétés cicatrisantes de la pectine

Dans cet exemple, on a testé les propriétés cicatrisantes de la pectine (E440) (Pectin Classic AU 201 USP par la société Herbstreith et Fox).

### 3.1 Principe du test et protocole

Le test de cicatrisation et d'invasion cellulaire (ou selon l'anglicisme : « *wound healing assay* ») consiste à créer une cicatrice ou brèche au niveau d'un tapis cellulaire de fibroblastes et à évaluer le temps nécessaire aux cellules disposées de part et d'autre de la cicatrice pour migrer et combler la brèche.

Il est basé sur l'étude de la cinétique de comblement de la brèche afin de suivre les processus de prolifération et de migration cellulaires participant au phénomène de la cicatrisation. L'analyse des images et la quantification des aires durant la fermeture de la cicatrice ont réalisées avec l'aide du logiciel de traitement et d'analyse d'images UTHSCSA Image Tool (version 3.0, Reindeer Graphics Inc., Asheville NC, USA).

Les fibroblastes humains proviennent de primocultures fournies par la société Biopredic sous la référence NHF (« *Normal Human Fibroblast »,* soit fibroblastes humains normaux).

Les fibroblastes ont été transférés, à une concentration de 1.10⁶ cellules/ml, dans une plaque de 24 puits (500 ml/puits) contenant les inserts (pièce en plastique positionnée dans les puits de culture cellulaire et servant de support de culture) et incubés à 37°C pendant une nuit. A la fin de la période d'incubation, les inserts ont été retirés des puits de culture cellulaire pour former des cicatrices et l'actif à tester y a été ajouté. Les cultures cellulaires ont ensuite été incubées à 37°C pendant 48H et le comblement des cicatrices a été suivi au microscope inversé muni d'un appareil photographique numérique.

Les images des fibroblastes (non représentées) ont été acquises au grossissement x100 après 48 heures d'incubation. Les résultats sont exprimés en pourcentages de comblement observé dans chaque brèche. Le taux d'induction est calculé à partir de la différence entre le taux de comblement observé dans l'échantillon et le taux de comblement obtenu dans le témoin négatif.

Les tests ont été effectués en utilisant une solution de pectine de pomme solubilisée dans le PBS et chauffée à 80°C pendant 30 minutes. Et celle-ci a été testées à différentes concentrations massiques : 0,01 % ; 0,05 % et 0,1 %.
Contrôle solvant : PBS
Contrôle positif : FGF (« *Fibroblast Growth Factor* »*,* soit facteur de croissance des fibroblastes) utilisé classiquement pour complémenter le milieu DMEM (10 ng/ml) : % du taux d'induction 24,13%

### 3.2. Résultats

Les résultats obtenus sont reportés dans le tableau 4 ci-après :

**TABLEAU 4**

| **Concentration de Pectine (% massique)** | **Taux d'induction (%)** |
|---|---|
| 0,01 | 13,81 |
| 0,05 | 8,69 |
| 0,1 | 0,75 |

Ces résultats mettent en évidence que la pectine possède des propriétés cicatrisantes et que le meilleur taux d'induction est obtenue avec une concentration massique de 0,01 %, ce taux ayant ensuite tendance à diminuer au fur et à mesure que la concentration massique en pectine augmente.

### EXEMPLE 4 : Préparation d'une composition comprenant de la pectine et un extrait de Centella asiatica

Une composition à base de pectine de pomme et d'extrait de *Centella asiatica* sous la forme d'une émulsion huile-dans-eau a été préparée comme indiqué ci-après.

Les pourcentages donnés ci-après sont des pourcentages massiques.

### Phase aqueuse (Phase A) :

- Eau (QSP) 100%
- Glycérine 13 %
- Stéarate de sucrose vendu sous la dénomination commerciale Surfhope® C-1816 par la société Gattefossé 3 %
- Pectine de pomme vendue sous la dénomination commerciale Pectin Classic AU 201 USP par la société Herbstreith et Fox 0,2 %
- Extrait de *Centella asiatica* vendu sous la dénomination commerciale *Centella asiatica* ES par la société Cooper 0,1 %

### Phase huileuse (Phase B)

- Triglycéride d'acides caprique et caprylique vendu sous la Dénomination commerciale DUB MCT par la société Verfilco 20 %
- Dipélargonate de Propylène Glycol vendu sous la dénomination commerciale DPPG CG par la société Gattefossé 3 %
- Squalane vendu sous la dénomination commerciale Phytosqualan par la société Sophim 2 %

### Phase C

- Copolymère Hydroxyéthyle Acrylate / Sodium Acryloyldiméthyle Taurate vendu sous la dénomination Sepinov EMT10 par la société Seppic 2 %
- Conservateurs q.s.
- Ajusteur de pH q.s. pH 6,00

Le protocole de préparation de cette émulsion est donné ci-après :

Phase A. Dans une première étape, et à température ambiante, incorporer la glycérine, la pectine, la *Centella asiatica* et le stéarate de sucrose. Mélanger le tout sous une agitation de 800-1000 Tr/min. Lorsqu'un mélange homogène est obtenu, incorporer l'eau QSP et chauffer cette phase A aux alentours de 80°C.

Phase B. Dans une deuxième étape, incorporer les huiles : soit le triglycéride d'acides caprique et caprylique, le dipélargonate de propylène glycol et le Squalane dans un autre contenant. Faire chauffer cette phase B aux alentours de 80°C.

Mettre la phase A sous agitation à 1500-2000 Tr/min.

Incorporer la phase B dans la phase A sous agitation. Laisser agiter 10 min à 80°C.

Phase C. Dans une troisième étape, incorporer la phase C sous agitation (1500 Tr/min) : le copolymère Hydroxyéthyle Acrylate / Sodium Acryloyldiméthyle Taurate, agiter 10 min pour laisser le temps à la formule de se gélifier. Refroidir le mélange toujours sous agitation jusqu'à température ambiante. Incorporer par la suite les conservateurs. Laisser agiter 15 min à température ambiante. Ajuster le pH.

On a ainsi obtenu une crème homogène dont le pH était voisin de 6.

## Revendications

1. Composition cosmétique ou pharmaceutique à application topique comprenant, dans un support cosmétiquement ou pharmaceutiquement acceptable, au moins une pectine en une quantité variant de 0,05 à 0,5 % en masse par rapport à la masse totale de la composition et au moins un extrait de *Centella asiatica* en une quantité variant de 0,1 à 1 % en masse par rapport à la masse totale de la composition.

2. Composition selon la revendication 1, **caractérisée en ce que** la pectine est une pectine de pomme.

3. Composition selon la revendication 1 ou 2, **caractérisée en ce qu'**elle présente un pH variant de 3 à 8.

4. Composition selon l'une quelconque des revendications précédentes, **caractérisée en ce qu'**elle comprend en outre au moins une phase grasse.

5. Composition selon la revendication 4, **caractérisée en ce que** la phase grasse comprend une ou plusieurs huiles choisies parmi les silicones volatiles ou non volatiles, linéaires, ramifiées ou cycliques, organo-modifiées ou non, hydrosolubles ou liposolubles ; les huiles minérales ; les huiles animales ; les huiles synthétiques ; les esters d'acides gras ; les huiles fluorées et perfluorées ; les cires ; les acides gras et les alcools gras.

6. Composition selon la revendication 4 ou 5, **caractérisée en ce que** la phase grasse représente de 1 à 80 % en masse par rapport à la masse totale de la composition.

7. Composition selon l'une quelconque des revendications précédentes, **caractérisée en ce qu'**elle se présente sous la forme d'une émulsion huile-dans-eau.

8. Composition selon l'une quelconque des revendications précédentes, **caractérisée en ce qu'**elle comprend un ou plusieurs principes actifs secondaires choisis parmi les agents desquamants, les agents hydratants, les agents anti-séborrhéiques, les agents dépigmentants ou propigmentants, les agents anti-glycation, les inhibiteurs de la NO-synthase, les inhibiteurs de la 5α-réductase, les agents stimulant la synthèse de macromolécules dermiques ou épidermiques et/ou empêchant leur dégradation, les agents stimulant la prolifération des fibroblastes ou des kératinocytes et/ou la différenciation des kératinocytes, les agents myorelaxants, les agents tenseurs, les agents anti-pollution ou anti-radicalaires, les agents apaisants, les actifs lipolytiques, ainsi que les agents agissant sur la microcirculation ou sur le métabolisme des cellules.

9. Utilisation non-thérapeutique d'une composition cosmétique à application topique telle que définie à l'une quelconque des revendications précédentes, pour le nettoyage, le démaquillage et/ou les soins de la peau, ou bien encore à titre de déodorant ou pour les soins d'hygiène corporelle.

10. Procédé cosmétique non thérapeutique de nettoyage et/ou de démaquillage de la peau comprenant l'application topique d'une composition cosmétique telle que définie à l'une quelconque des revendications 1 à 8, sur les zones de la peau concernées.

11. Composition pharmaceutique à application topique comprenant, dans un support pharmaceutiquement acceptable, au moins une pectine en une quantité variant de 0,05 à 0,5 % en masse par rapport à la masse totale de la composition et au moins un extrait de *Centella asiatica* en une quantité variant de 0,1 à 1 % en masse par rapport à la masse totale de la composition, pour une utilisation dans la prévention et/ou le traitement des affections cutanées impliquant la présence de microorganismes à la surface de la peau, en particulier la prévention et/ou le traitement des escarres et des lésions ischémiques.

## Patentansprüche

1. Kosmetische oder pharmazeutische Zusammensetzung für topische Anwendung, umfassend, in einem kosmetisch oder pharmazeutisch akzeptablen Träger, mindestens ein Pektin in einer Menge, die von 0,05 bis 0,5 Ma% in Bezug zur Gesamtmasse der Zusammensetzung schwankt, und mindestens einen Extrakt aus *Centella asiatica* in einer Menge, die von 0,1 bis 1 Ma% in Bezug zur Gesamtmasse der Zusammensetzung schwankt.

2. Zusammensetzung nach Anspruch 1, **dadurch gekennzeichnet, dass** das Pektin ein Apfelpektin ist.

3. Zusammensetzung nach Anspruch 1 oder 2, **dadurch gekennzeichnet, dass** sie einen pH aufweist, der von 3 bis 8 schwankt.

4. Zusammensetzung nach einem der vorangehenden Ansprüche, **dadurch gekennzeichnet, dass** sie ferner mindestens eine Fettphase umfasst.

5. Zusammensetzung nach Anspruch 4, **dadurch gekennzeichnet, dass** die Fettphase ein oder mehrere Öle umfasst, die aus den volatilen oder nicht volatilen, linearen, verzweigten oder cyclischen, organomodifizierten oder nicht, wasserlöslichen oder fettlöslichen Silikonen; den Mineralölen; den tierischen Ölen; den synthetischen Ölen; den Fettsäureestern; den fluorierten und perfluorierten Ölen; den Wachsen; den Fettsäuren und den Fettalkoholen ausgewählt sind.

6. Zusammensetzung nach Anspruch 4 oder 5, **dadurch gekennzeichnet, dass** die Fettphase 1 bis 80 Ma% in Bezug zur Gesamtmasse der Zusammensetzung darstellt.

7. Zusammensetzung nach einem der vorangehenden Ansprüche, **dadurch gekennzeichnet, dass** sie sich in Form einer Öl-in-Wasser-Emulsion darstellt.

8. Zusammensetzung nach einem der vorangehenden Ansprüche, **dadurch gekennzeichnet, dass** sie einen oder mehrere sekundäre Wirkprinzipien umfasst, ausgewählt aus den abschuppenden Wirkstoffen, den feuchtigkeitsspendenden Wirkstoffen, den antiseborrhoischen Wirkstoffen, den depigmentierenden oder propigmentierenden Wirkstoffen, den Antiglycationswirkstoffen, den Inhibitoren der NO-Synthase, den Inhibitoren der 5α-Reduktase, den Wirkstoffen, die die Synthese von dermischen oder epidermischen Makromolekülen stimulieren und/oder ihren Abbau verhindern, den Wirkstoffen, die die Weiterverbreitung der Fibroblasten oder der Ketatinozyten und/oder die Differenzierung der Keratinozyten stimulieren, den muskelentspannenden Wirkstoffen, den straffenden Wirkstoffen, den Wirkstoffen gegen Verschmutzung oder Radikale, den reizlindernden Wirkstoffen, den fettlösenden Wirkstoffen sowie den Wirkstoffen, die die Mikrozirkulation oder den Stoffwechsel der Zellen beeinflussen.

9. Nicht-therapeutische Verwendung einer kosmetischen Zusammensetzung für topische Anwendung nach einem der vorangehenden Ansprüche für die Reinigung, das Abschminken und/oder die Pflege der Haut oder auch als Deodorant oder zur Hygiene und Pflege des Körpers.

10. Nicht-therapeutisches kosmetisches Reinigungs- und/oder Abschminkverfahren der Haut, umfassend die topische Anwendung einer kosmetischen Zusammensetzung nach einem der Ansprüche 1 bis 8 auf den betroffenen Hautbereichen.

11. Pharmazeutische Zusammensetzung für topische Anwendung, umfassend, in einem pharmazeutisch akzeptablen Träger, mindestens ein Pektin in einer Menge, die von 0,05 bis 0,5 Ma% in Bezug zur Gesamtmasse der Zusammensetzung schwankt und mindestens einen Extrakt aus *Centella asiatica* in einer Menge, die von 0,1 bis 1 Ma% in Bezug zur Gesamtmasse der Zusammensetzung schwankt, für eine Verwendung bei der Vorbeugung und/oder der Behandlung von Beeinträchtigungen der Haut bei Anwesenheit von Mikroorganismen auf der Oberfläche der Haut, insbesondere der Vorbeugung und/oder der Behandlung von Dekubiti und Läsionen ischämischen Charakters.

## Claims

1. Cosmetic or pharmaceutical composition for topical application comprising, in a cosmetically or pharmaceutically acceptable carrier, at least one pectin in an amount varying from 0.05 to 0.5 weight % relative to the total weight of the composition and at least one extract of *Centella asiatica* in an amount varying from 0.1 to 1 weight % relative to the total weight of the composition.

2. The composition according to claim 1, **characterized in that** the pectin is apple pectin.

3. The composition according to claim 1 or 2, **characterized in that** it has a pH varying from 3 to 8.

4. The composition according to any of the preceding claims, **characterized in that** it further comprises at least one fat phase.

5. The composition according to claim 4, **characterized in that** the fat phase comprises one or more oils selected from among silicones that are volatile or non-volatile, straight-chain, branched or cyclic, organomodified or non-organomodified, water-soluble or fat-soluble; mineral oils; animal oils; synthetic oils; fatty acid esters; fluorinated and perfluorinated oils; waxes; fatty acids and fatty alcohols.

6. The composition according to claim 4 or 5, **characterized in that** the fat phase represents 1 to 80 % by weight relative to the total weight of the composition.

7. The composition according to any of the preceding claims, **characterized in that** it is in the form of an oil-in-water emulsion.

8. The composition according to any of the preceding claims, **characterized in that** it comprises one or more secondary active ingredients selected from among desquamating agents, hydrating agents, anti-seborrhoea agents, depigmenting or pro-pigmenting agents, anti-glycation agents, NO-synthase inhibitors, 5α-reductase inhibitors, agents stimulating the synthesis of dermal or epidermal macromolecules and/or preventing the degradation thereof, agents stimulating the proliferation of fibroblasts or keratinocytes and/or keratinocyte differentiation, myorelaxant agents, tensing agents, anti-pollution or anti-radical agents, soothing agents, lipolytic active substances, and agents acting on microcirculation or on cell metabolism.

9. Non-therapeutic use of a cosmetic composition for topical application such as defined in any of the preceding claims, for skin cleansing, makeup removal and/or skincare, or as deodorant or for body hygiene care.

10. Non-therapeutic cosmetic method for skin cleansing and/or makeup removal, comprising the topical application of a cosmetic composition such as defined in any of claims 1 to 8, to the skin regions concerned.

11. Pharmaceutical composition for topical application comprising, in a pharmaceutically acceptable carrier, at least one pectin in an amount varying from 0.05 to 0.5 weight % relative to the total weight of the composition, and at least one extract of *Centella asiatica* in an amount varying from 0.1 to 1 % by weight relative to the total weight of the composition, for use in the prevention and/or treatment of skin disorders involving the presence of microorganisms on the surface of the skin, in particular the prevention and/or treatment of bedsores and ischaemic lesions.
